# EUROPEAN PATENT SPECIFICATION

(11) **EP 2 424 598 B1**
(45) Date of publication and mention of the grant of the patent: **11.12.2013**
(21) Application number: 10717101.9
(22) Date of filing: 21.04.2010
(51) Int. Cl.: A61M 5/32

(54) **Needle remover and method for removing a needle**
Vorrichtung zur Entfernung einer Nadel und Verfahren zur Entfernung einer Nadel
Suppression d'aiguille et procédé de suppression d'une aiguille

(30) Priority: 30.04.2009 EP 09005997
(43) Date of publication of application: 07.03.2012
(73) Proprietor: Sanofi-Aventis Deutschland GmbH, 65929 Frankfurt (DE)
(72) Inventor: BODE, Andreas, 65926 Frankfurt am Main (DE)
(86) International application number: PCT/EP2010/055254
(87) International publication number: WO 2010/124974

(56) References cited:
- EP-A- 1 598 043
- WO-A-2005/079889
- US-A- 5 573 113
- US-A1- 2005 288 363

## Description

The invention relates to a needle remover and a method for removing a needle from a holder.

Use of injection devices in hospitals, elderly homes or by any third person bears the risk of infection by needle stick injuries. A user may receive a needle stick injury during removal of a needle from an injection device. A conventional needle remover is held with one hand while the other hand holds the device. The operation of this needle remover involves the risk that the needle is stuck into the hand which holds the needle remover during needle removal.

Detaching the needle after use is a dangerous operation with respect to needle stick injuries. This hazard can be averted if the user does not touch the needle.

Document US 5,409,113 shows a disposal container for automatic release of needles from a holder and for disposal of the needles. The disposal container has a release aperture formed in the top, the aperture including opposite tapered sides extending into the container. The needle is mounted to a needle holder, the holder having a depressible lock element extending from the holder. The holder is inserted in the release aperture, with the lock element engaging the tapered side, and the holder is then depressed to eject the needle automatically from the holder.

Document WO 90/03315 shows an apparatus for safely removing needles from a syringe, which requires only one hand. The user inserts the syringe into a throat of a needle guiding house and presses downward, whereupon the needle guiding house moves downward, disconnecting gear teeth engaging the collar of the needle. The disconnecting gear teeth unscrew the needle and allow it to drop into a container.

Document US 5 573 113 A discloses a needle removal apparatus comprising a support member and an elongated tubular sleeve, wherein said sleeve is reciprocally mounted in a throughbore of the support member. On an inner end of said sleeve, a coupling means is provided for direct coupling to a needle hub for unthreading a needle hub from an end of a tubular barrel during movement with said sleeve toward an innermost position.

It is an aim of the present invention to provide an alternative apparatus for safely removing needles from a holder.

For this aim a needle remover for removing a needle from a holder is provided. The needle remover comprises a guiding member which is suitable for receiving the needle, and a gripping means which is suitable for gripping the needle when the needle is at least partly inserted in the guiding member in an inserting direction. The gripping means is configured to rotate when the gripping means is moved in the inserting direction with respect to the guiding member so that the gripped needle is unscrewed from the holder when the holder is moved in the inserting direction with respect to the guiding member.

The holder and the needle move in the same direction longitudinally but the needle also follows a rotational movement. When the user moves the holder longitudinally so that rotational movement of the holder with respect to the guiding means is prevented or almost prevented, torque impacts to needle thereby unscrewing the needle. In other words, the user holds the holder so that it does not or almost not rotate during its longitudinal movement in order to provide counter acting torque. The torque is provided when the gripping means which hold the needle rotate with respect to the holder.

In an alternative embodiment the guiding means is designed so that rotational movement of the holder in prevented. In one embodiment the holder has a profile which is not circular and an opening of the guiding means corresponds to the profile of the holder, so that the rotational movement of the holder with respect to the guiding means is prevented. In one embodiment one of the opening and the holder comprises longitudinally extending ribs and the other one comprises longitudinally extending trenches. In a further embodiment a component is provided which prevents rotational movement of the holder with respect to the guiding means. This component may be designed as attachment which is mounted to the needle remover or guiding means, for example.

The needle remover allows removing the needle with one hand only, therefore reducing the hazard of needle stick injuries. One hand holds the holder but the needle is gripped by the needle remover.

The needle comprises a cannula and a needle collar which is suitable to releasably engage the needle with the holder. In one embodiment the needle is detachable from the holder by unscrewing. The collar may be designed as screw connector.

The holder may be formed as drug delivery device wherein the drug is delivered through the cannula of the needle. Preferably disposable needles are used with non-disposal drug delivery devices. The drug delivery device may be designed as pen-type injection device or syringe, for example. In one embodiment the holder is designed as device which is suitable to take a sample of blood from the patient. Such a device may be designed as syringe.

The guiding member is designed to at least partly receive the needle when the holder is partly inserted into the guiding member into an inserting direction.

The inserting direction with respect to the needle remover is the direction in which the holder is moved so that the needle is inserted into the guiding member.

The gripping means is designed to grip the needle. In one embodiment the needle is held by a clamping connection. The needle may be squeezed into the gripping means. In an alternative embodiment the needle releasably engages with the gripping means.

The gripping means is designed to move with respect to the guiding member so that the gripped needle is unscrewed from the holder. The gripping means rotates with respect to the guiding member when the gripping means is moved in the inserting direction, thereby rotating the gripped needle with respect to the holder so that the needle is unscrewed.

The guiding member of the needle remover comprises a helical structure. The helical structure serves as guide for the gripping means. The gripping means can be moved in the inserting direction along the helical structure. In one embodiment the helical structure is designed as thread.

The gripping means is configured to engage with the helical structure so that the gripping means is moveable along the helical structure. The gripping means is configured to hold the needle in a force-locked manner.When force is applied to the gripping means in the inserting direction, the gripping means rotates during movement in the inserting direction. Force may be applied by moving the holder into the inserting direction,with respect to the guiding member. This force is transferred to the gripping means by means of the gripped needle, thereby rotating the gripping means during its movement in the inserting direction so that the gripped needle is unscrewed from the holder when the holder is moved in the inserting direction with respect to the guiding member.

In one embodiment of the needle remover, the gripping means is suitable for releasing the needle after unscrewing the needle. The unscrewed needle is automatically released at the end of the needle removal operation, which prevents needle stick injuries.

In one embodiment the gripping means is designed to hold the needle in a force-locked manner, which is a frictional or non-positive connection. In one embodiment the gripping means comprises an elastic gripping lining. The needle is squeezed into the elastic gripping lining, thereby deforming the gripping lining so that the needle is held. Rotational movement of the needle for latching is not necessary. However, in an alternative embodiment the gripping means is designed to hold the needle with a positive locking, for example by means of a snapping connection.

In one embodiment the gripping means comprises a first clamping member and a second clamping member, which are moveable with respect to one another. The first and second clamping members are moveable with respect to one another so that they can hold the needle when they are in one position. In other words, the first clamping member is positioned in a first position with respect to the second clamping member if the needle is gripped. The needle is released when the first and second clamping members are moved to another position. In other words, the needle is released when the first clamping member is positioned in a second position with respect to the second clamping member.

First position means that the first clamping member is positioned with respect to the second clamping member so that they can grip the needle. Second position means that the first clamping member is positioned with respect to the second clamping member so that the needle is released. The first clamping member moves radially outwards with respect to the helical structure during movement from the first position to the second position.

In one embodiment the guiding member comprises a first sleeve having a helical cut-out which serves as guide for the gripping member. The sleeve may be formed as cylinder. The gripping member is moveable along the helical cut-out. In an alternative embodiment the first sleeve has more than one helical cut-out, wherein the cut-outs are positioned with angular offsets.

In one embodiment the first clamping member comprises a jaw member that is located inside the first sleeve and a protruding member which protrudes from the first sleeve. The connection, which is formed as arm for example, between the jaw member and the protruding member extends through the helical cut-out. The first clamping member is radially moveable with respect to the first sleeve. However, the jaw member and the protruding member are formed so that they cannot move through the cut-out, which limits the radial movement of the first clamping member.

The jaw member is suitable for holding the needle to prevent movement of the needle with respect to the jaw member through inward pressure. In one embodiment the gripping means comprises a frame and a jaw member which is moveable with respect to the frame in order to hold the needle between the jaw member and the frame or to release the needle. This arrangement may look similar to a G-clamp. The gripping means is moveable along the helical cut-out so that the gripping means rotates during movement into the inserting direction, which causes the needle to be unscrewed.

Preferably, the second clamping member also comprises a jaw member. When the first clamping is positioned in the first position with respect to the second clamping member the clamping members are arranged so that their jaw members can hold the needle. The first and second clamping members are moveable along the helical cut-outs if the first clamping member is positioned in the first position with respect to the second clamping member so that the first and second clamping members rotate, which causes the needle to be unscrewed. When the first clamping member is moved radially outwards with respect to the helical cut-out so that the distance between the jaw members is increased, a gripped needle is released.

One embodiment comprises a biasing member which acts normally to urge the first clamping member in the second position, in which the needle is released. In one embodiment the biasing member is coupled between the first sleeve and the protruding member of the first clamping member. The biasing member is deformable so that the first clamping member can be moved to the first position, in which the needle can be gripped. One embodiment of the biasing member is designed as spring.

One embodiment of the needle remover comprises a moveable pushing member. The pushing member is designed to push the first clamping member into the first position when the pushing member is positioned in a pushing position. The biasing member is deformed when the first clamping member is pushed. When the pushing member is positioned in the release position, the pushing member does not block radial outward movement of the first clamping member. The biasing member pushes the first clamping member into the second position.

In one embodiment the first sleeve is located at least partly inside a second sleeve so that the first clamping member is positioned in the first position when the gripping means is moved in the inserting direction. The side wall of the second sleeve limits the radial movement of the first clamping member so that the first clamping member is forced into the first position when its protruding member moves between the first and second sleeves in the inserting direction. The arrangement of the second sleeve ensures that the needle is held during longitudinal movement of the clamping members.

Preferably a bin comprises a needle reservoir which is suitable for receiving unscrewed needles and the needle remover. The bin serves as disposal container. The combination of the needle remover and the container ensures that the needles drop into the container after removal. Removing and dropping the needle is a one-hand operation. The needle is unscrewed und dropped automatically when the holder is inserted into the needle remover, which prevents needle stick injuries.

One embodiment of the bin comprises a lid which is suitable to cover an opening of the guiding member, wherein the lid is coupled with the pushing member so that the pushing member is positioned in the release position when the lid covers the opening. The pushing member moves to the pushing position when the lid is removed from the opening. Thus, the first clamping member is positioned in the first position with respect to the second clamping member, ready to hold the needle, when the lid is removed from the opening. The first and second clamping members release the needle when the opening is closed. In one embodiment the lid and the pushing member are integrally formed. In an alternative embodiment the pushing member and the lid are connected via a hinge.

A method for removing a needle from a holder by a needle remover comprises the following steps. The needle is inserted in an inserting direction into a guiding member of the needle remover. Then the needle is gripped by a gripping means when the needle is at least partly inserted in the guiding member. The gripping means rotates when the gripping means is moved in the inserting direction with respect to the guiding member so that the gripped needle is unscrewed from the holder when the holder is moved in the inserting direction with respect to the guiding member.

Further features and refinements become apparent from the following description of the exemplary embodiments in connection with the accompanying figures.
Figure 1 shows an embodiment of a bin having a needle remover in a needle removing state.
Figure 2 shows the embodiment of a bin having a needle remover in an initial state.
Figure 3 shows a side view of an embodiment of a first clamping member and an embodiment of a second clamping member.
Figure 4 shows the top view of an embodiment of a first clamping member and an embodiment of a second clamping member.
Figure 5 shows a first sleeve and the first and second clamping member of an embodiment of the needle remover in detail.
Figure 6 shows the needle gripped by the first and second clamping members in detail.
Figure 7 shows the needle released by the first and second clamping members in detail.

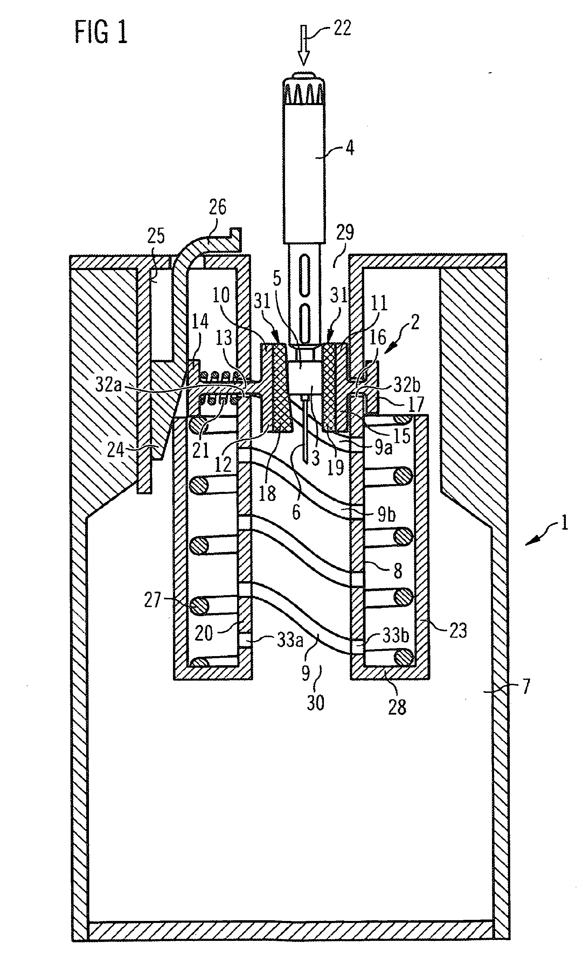

Figure 1 shows an embodiment of a bin having a needle remover.

The bin 1 comprises a needle remover 2 which is suitable for removing the needle 3 from a holder 4. In this embodiment the holder 4 is designed as injection pen.

The injection pen is expediently adapted for administering a medicament.

The term "medicament", as used herein, preferably means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, a antibody, an enzyme, an antibody, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exedin-3 or exedin-4 or an analogue or derivative of exedin-3 or exedin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(826) human insulin; Des(B28-B30) human insulin; Des(B27) human insulin and Des(B30) human insulin.

Insulin derivates are for example B29-N-myristoyl-des(B30) human insulin; B29-N-palmitoyl-des(830) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
   H-(Lys)5-des Pro36, des Pro37 Exendin-4(1-39)-NH2,
   des Pro36 [Asp28] Exendin-4(1-39),
   des Pro36 [IsoAsp28] Exendin-4(1-39),
   des Pro36 [Met(O)14, Asp28] Exendin-4(1-39),
   des Pro36 [Met(O)14, IsoAsp28] Exendin-4(1-39),
   des Pro36 [Trp(O2)25, Asp28] Exendin-4(1-39),
   des Pro36 [Trp(O2)25, IsoAsp28] Exendin-4(1-39),
   des Pro36 [Met(O)14 Trp(O2)25, Asp28] Exendin-4(1-39),
   des Pro36 [Met(O)14 Trp(O2)25, IsoAsp28] Exendin-4(1-39); or
des Pro36 [Asp28] Exendin-4(1-39),
   des Pro36 [IsoAsp28] Exendin-4(1-39),
   des Pro36 [Met(O)14, Asp28] Exendin-4(1-39),
   des Pro36 [Met(O)14, IsoAsp28] Exendin-4(1-39),
   des Pro36 [Trp(O2)25, Asp28] Exendin-4(1-39),
   des Pro36 [Trp(O2)25, IsoAsp28] Exendin-4(1-39),
   des Pro36 [Met(O)14 Trp(O2)25, Asp28] Exendin-4(1-39),
   des Pro36 [Met(O)14 Trp(O2)25, IsoAsp28] Exendin-4(1-39),
wherein the group -Lys6-NH2 may be bound to the C-terminus of the Exendin-4 derivative;
or an Exendin-4 derivative of the sequence
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(O2)25, Asp28] Exendin-4(1-39)-Lys6-NH2,
H-des Asp28 Pro36, Pro37, Pro38 [Trp(O2)25] Exendin-4(1-39)-NH2,
H-(Lys)6-des Pro36, Pro37, Pro38 [Trp(O2)25, Asp28] Exendin-4(1-39)-NH2,
H-Asn-(Glu)5-des Pro36, Pro37, Pro38 [Trp(O2)25, Asp28] Exendin-4(1-39)-NH2,
des Pro36, Pro37, Pro38 [Trp(O2)25, Asp28] Exendin-4(1-39)-(Lys)6-NH2,
H-(Lys)6-des Pro36, Pro37, Pro38 [Trp(O2)25, Asp28] Exendin-4(1-39)-(Lys)6-NH2,
H-Asn-(Glu)5-des Pro36, Pro37, Pro38 [Trp(O2)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(O2)25, Asp28] Exendin-4(1-39)-Lys6-NH2,
H-des Asp28 Pro36, Pro37, Pro38 [Met(O)14, Trp(O2)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(O2)25, Asp28] Exendin-4(1-39)-NH2,
des Pro36, Pro37, Pro38 [Met(O)14, Trp(O2)25, Asp28] Exendin-4(1-39)-(Lys)6-NH2,
H-(Lys)6-des Pro36, Pro37, Pro38 [Met(O)14, Trp(O2)25, Asp28] Exendin-4(S1-39)-(Lys)6-NH2,
H-Asn-(Glu)5-des Pro36, Pro37, Pro38 [Met(O)14, Trp(O2)25, Asp28] Exendin-4(1-39)-(Lys)6-NH2;
or a pharmaceutically acceptable salt or solvate of any one of the afore-mentioned Exedin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

The needle 3 comprises a needle collar 5 and a cannula 6. The needle 3 is detachable from the holder 4 by unscrewing it, which means that the needle 3 is rotated with respect to the needle holder 4. The needle collar 5 may be designed as screw connector.

In one embodiment the holder 4 comprises an outer thread (not shown) and the needle collar 4 comprises an inner thread (not shown) which is releasably connectable with the outer thread of the holder 4.

The bin 1 further comprises a reservoir 7 which is suitable for receiving unscrewed needles 3. The needle remover 2 may be mounted to a mouth of a container which serves as reservoir 7. The container may be made of glass or plastic, for example. In one embodiment the reservoir and at least parts of the needle remover are integrally formed.

The needle remover 2 comprises a guiding member 8 being suitable for receiving the needle 3. The guiding member 8 is formed as first sleeve 20 which has a top opening 29 and a bottom opening 30. The holder 4 with the needle 3 is to be inserted in an inserting direction 22 through the top opening 29.

The guiding member 8 comprises a helical structure 9 which comprises a first helical cut-out 9a and a second helical cut-out 9b. The first helical cut-out 9a has an angular offset of 180 degree with respect to the second helical cut-out 9b.

A gripping means 31 which is suitable for holding the needle 3 comprises a first camping member 10 and a second clamping member 11. The first clamping member 10 comprises a first jaw member 12 which has a gripping lining 18, and a protruding member 14, the first jaw member 12 and the first protruding member 14 being connected by a first arm 13. The second clamping member 11 comprises a second jaw member 15 which has a second gripping lining 19, the second jaw member 15 and the second protruding member 17 being connected by a second arm 16.

The first clamping member 10 is arranged so that the first jaw member 12 is located inside the first sleeve 20 and so that the protruding member 14 protrudes from the first sleeve 20. The arm 13 runs through the first cut-out 9a. A first spring 21 is positioned around the first arm between the first protruding member 14 and the first sleeve 20. The first spring 21 is formed as compression coil spring which is designed to resist being compressed. The first spring 21 serves as biasing member of a spring loaded mechanism suitable for locking and releasing the needle.

The first arm 13 is moveable along the first cut-out 9a so that the first clamping member 10 is moveable along the first helical cut-out 9a. In one embodiment the first arm 13 mainly has a circular profile. In an alternative embodiment the profile of the first arm 13 is mainly rectangular, for example. A non-circular profile is suitable for preventing rotational movement of the first arm 13 with respect to the first cut-out 9a.

The first arm 13 can be radially moved through the first cut-out 9a with respect to the first sleeve 20. The first protruding member 14 and the first jaw member 12 are designed so that they cannot be moved through the first cut-out 9, which limits the radial movement of the first clamping member 10. Radial inward movement of the first clamping member 10 with respect to the first sleeve 20 is stopped when the first spring 21 is totally compressed. The radial outward movement with respect to the first sleeve 20 is stopped when first jaw member 12 reaches the first sleeve 20.

The second camping member 11 is arranged so that the second jaw member 15 is located inside the first sleeve 20 and so that the second protruding member 17 protrudes from the first sleeve 20. The second arm 16 runs through the second helical cut-out 9b.

The arm 16 can be moved along the second helical cut-out 9b. Thus, the second clamping member 11 can be moved along the second helical cut-out 9b. In one embodiment the arm 16 mainly has a circular profile. In an alternative embodiment the profile of the arm 16 is mainly rectangular, for example. The second protruding member 17 and second jaw member 15 are designed so that they cannot be moved through the second helical cut-out 9b.

In this embodiment the second clamping member 11 is not or almost not radially moveable with respect to the first sleeve 20, because the length of the second arm 16 corresponds to the thickness of the wall of the first sleeve 20. However, in an alternative embodiment (not shown) the second clamping member 11 can be radially moved with respect to the first sleeve 20.

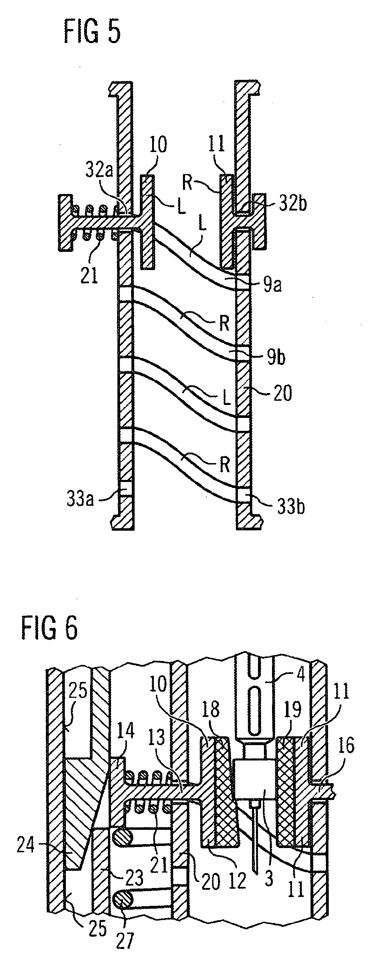

Figure 5 shows the helical cut-outs 9a, 9b and the first sleeve 20 of an embodiment in detail. The first and second clamping members 10, 11 are located at the top ends 32a, 32b of the helical cut-outs 9a, 9b. Each helical cut-out 9a, 9b turns twice around the first sleeve 20 so that clamping members 10, 11 rotate around 720 degrees on their way from the top ends 32a, 32b to the bottom ends 33a, 33b of the helical cut-outs 9a, 9b. This rotation is sufficient for unscrewing the needle 3 from the holder 4 (not shown in figure 5).

Referring to figure 1 again, the first and second clamping members 10, 11 serve as gripping means being suitable for gripping the needle 3 when the needle 3 is at least partly inserted into the first sleeve 20.

In a first position the first clamping member 10 is positioned with respect to the second clamping member 11 so that the first spring 21 is at least partly compressed. The first and second clamping members 10, 11 are positioned so that the needle collar 5 can be held between the first and second gripping linings 18, 19. In a second position the first clamping member 10 is positioned with respect to the second clamping member 11 so that the first spring 21 is relaxed or at least more relaxed than in the first position of the first clamping member 10.

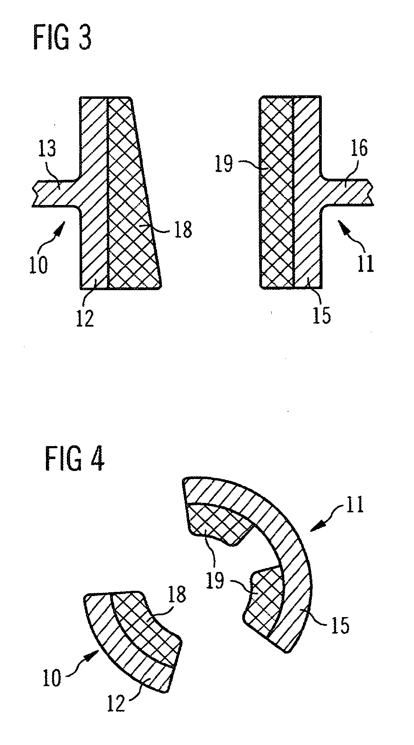

Figure 3 shows a side view of one embodiment of the first and second clamping members 10, 11. The first and second gripping linings 18, 19 are made of an elastic material. In one embodiment they comprise rubber, for example. The first gripping lining 18 is sloped, which facilitates moving the needle collar (not shown in figure 3) between the first and second gripping lining 18, 19 until the needle collar 5 is squeezed between the first and second gripping lining 18, 19 by the deformation of the elastic material.

Figure 4 shows a top view of one embodiment of the first and second clamping members 10, 11, which are mainly shaped as circular arc. The second clamping member 11 extends over a larger circular arc than the first clamping member 10. The first gripping lining 18 extends along the inside wall of the first clamping member 10. The second clamping member 11 comprises two gripping linings 19, which are mounted to the external ends of the inside wall of the second clamping member 11. This arrangement enables to clasp the needle collar 5 (not shown in figure 4) at three points so that the needle 3 is safely gripped. In one embodiment at least one of the gripping linings 18, 19 is sloped (not explicitly shown in figure 4).

Referring to figure 1 again, the first and second clamping members 10, 11 can be moved along the first and second helical cut-out 9a, 9b, respectively, from top ends 32a, 32b of the helical cut-outs 9a, 9b to the bottom ends 33a, 33b of the helical cut-outs 9a, 9b. Thereby the first and second clamping members 10, 11 rotate around a screw axis of the helical cut-outs 9a, 9b and move in the inserting direction 22.

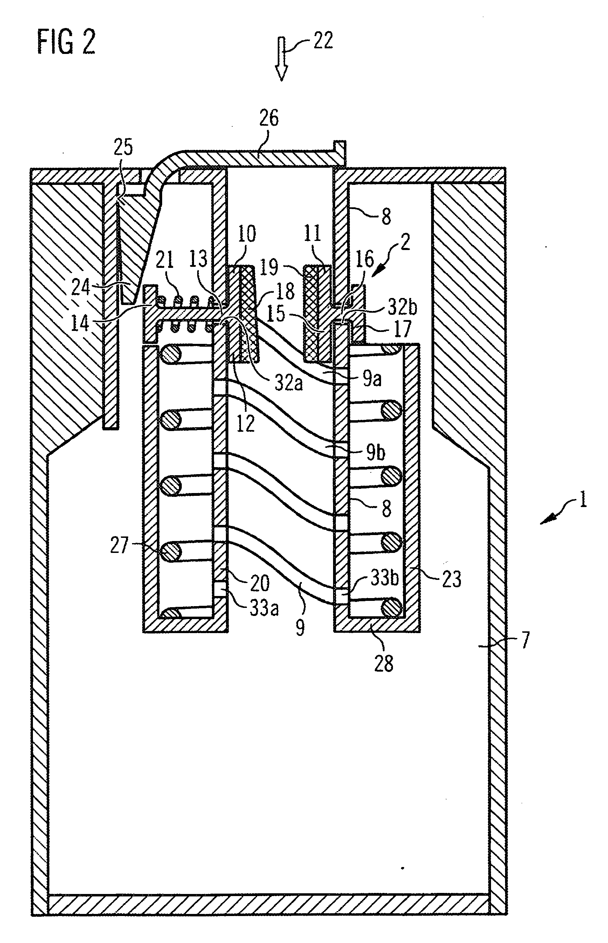

A pushing member 24 is provided which can be moved along a side wall 25 of the bin 1. The pushing member 24 can be moved from a release position (not shown in figure 1, but shown in figure 2) to a pushing position, as shown in figure 1, and vice versa. The pushing member 24 is designed as spline. In the pushing position, the spline pushes towards the first protruding member 14 of the first clamping member 10 which is located at the top end 32a of the helical cut-out 9a so that the first spring 21 is compressed. Thereby, the clamping member 10 is positioned in the first position with respect to the second clamping member 2 so that clamping members 10, 11 can grip the needle 3.

When the pushing member 24 is positioned in the release position, the pushing member no longer blocks radial outward movement of the first clamping member 10 which is located at the top end 32a of the helical cut-out 9a any more so that the first spring 21 forces the first camping member 10 to move radially outwards with respect to the first sleeve 20 to the second position.

The pushing member 24 is connected with a lid 26. When the lid 26 is pulled out of the bin 1, the pushing member 24 is moved from the pushing position to the release position. When the lid 26 is pulled into the bin 1, the pushing member 24 is moved from the release position to the pushing position.

The flexible lid 26 is suitable for covering the top opening 29 of the first sleeve 20, which serves as receiving opening for the needle 3. When a user pulls the lid 26 in order to cover the receiving opening 29, the pushing member 24 is moved from the pushing position to the release position. When the lid 26 is removed from the receiving opening 29, the lid is pulled into the bin 1, which causes the pushing member 24 to be moved into the push position.

In an alternative embodiment (not shows) the pushing member 24 is coupled with a button element which can be pressed by the user. In one embodiment the pushing member moves from the pushing position to the release position when the button element is pressed.

The bottom part of the first sleeve 20 is positioned in a second sleeve 23. The distance between the side walls of the first sleeve 20 and the second sleeve 23 is so dimensioned that the protruding member 14 of the first clamping member 10 can only move between the first and second sleeves 20, 23 if the first clamping member 10 is positioned in the first position.

A second spring 27 which is designed as compression coil spring is positioned between the side walls of the first and second sleeves 20, 23. The second spring 27 has a top part and a bottom part. A stop disc 28 is located between the bottom ends of the first and second sleeves 20, 23. The top end of the second spring 27 is located adjacent to the first and second clamping members 10, 11, and the bottom end of the second spring 27 is located adjacent to the stop disc 28. When the first and second clamping members 10, 11 are positioned at the top of the helical cut-outs 9a, 9b, the second spring is in a relaxed or almost relaxed state. When the first and second clamping members 10, 11 are moved in the inserting direction 22, the second spring 27 is compressed. In one embodiment the second spring is compressed by the protruding member 14, 17 of at least one of the first and second clamping members 10, 11. When the compressed second spring 27 relaxes the first and second clamping members 10, 11 are moved back to the top ends 32a, 32b of the helical cut-outs 9a, 9b.

Figure 2 shows the bin 1 in the initial position. The lid 26 covers the top opening 29 of the first sleeve 20, which serves as receiving opening for the needle 3 (not shown in figure 2). The pushing member 24 is positioned in the release position.

The first and second clamping members 10, 11 are located at the top ends 32a, 32b of the first and second helical cut-outs 9a, 9b. The first spring 21 pushes the first clamping member 10 into the first position. In this position the protruding member 14 extends beyond the inside wall of the second sleeve 23 so that the first clamping member 10 cannot move in the inserting direction 22.

Operation of the needle remover 2 is described as follows. Before operation the user removes the lid 26 from the receiving opening 29. The lid 26 is pulled into the bin 1, thereby moving the pushing member 24 from the release position to the pushing position.

When the spline-shaped pushing member 24 moves to the pushing position, the pushing member 24 pushes the first protruding member 14 of the first clamping member 10 radially inwards with respect the first sleeve 20. The first clamping member 10 is positioned in the first position, wherein the protruding member 14 no longer extends beyond the inside wall of the second sleeve 23.

The holder 4, to which the needle 3 is mounted, is moved through the receiving opening 29 into the first sleeve 20 so that the needle 3 is squeezed between the first and second gripping linings 18, 19 of the first and second clamping members 10, 11, respectively. Figure 6 shows the gripped needle 3 in detail. The needle 3 is guided to the gripping position by the sloped first gripping lining 18. The needle 3 is squeezed between the elastic first and second gripping linings 18, 19 so that the needle 3 can no longer be moved in the inserting direction 2 with respect to the clamping members 10, 11.

Figure 1 shows the operation state immediately after gripping the needle 3 and before starting to unscrew the needle 3.

When the needle holder 4 is further moved in the inserting direction 22, the first and second clamping members 10, 11, which grip the needle 3, are moved in the inserting direction 22 along the first and second helical cut-outs 9a, 9b, respectively. The first and second clamping members 10, 11 rotate when they are moved in the inserting direction 22 with respect to the first sleeve 20 so that the gripped needle 3 is unscrewed from the holder 4 when the holder 4 is moved in the inserting direction 22 with respect to the first sleeve 20.

When the first and second clamping members 10, 11 move from the top ends 32a, 32b to the bottom ends 33a, 33b of the first and second helical cut-outs 9a, 9b, respectively, they rotate around 720 degree, which is sufficient to unscrew the needle 3 from the holder 4.

The second spring 27 is compressed when the first and second clamping members 10, 11 move into the inserting direction. When the first and second clamping members 10, 11 reach the bottom ends 33a, 33b of the helical cut-out 9a, 9b, further movement in the inserting direction 22 is stopped. The holder 4 can be withdrawn. The unscrewed needle 3 is held by the first and second clamping members 10, 11.

After withdrawal of the holder 4, the second spring 27 relaxes, thereby forcing the first and second clamping members 10, 12 to move in the direction opposite to inserting direction 22. The first spring 21 is compressed during this movement, because the inside wall of the second sleeve 23 stops the radial outward movement of the first clamping member 10.

When the first and second clamping members 10, 12 reach the top ends 32a, 32b of the first and second helical cut-outs 9a, 9b, their movement in the direction opposite to inserting direction 22 is stopped. The pushing member 24, which is positioned in the pushing position, blocks radial outward movement of the first clamping member 10 so that the needle 3 is still held by the first and second clamping members 10, 11.

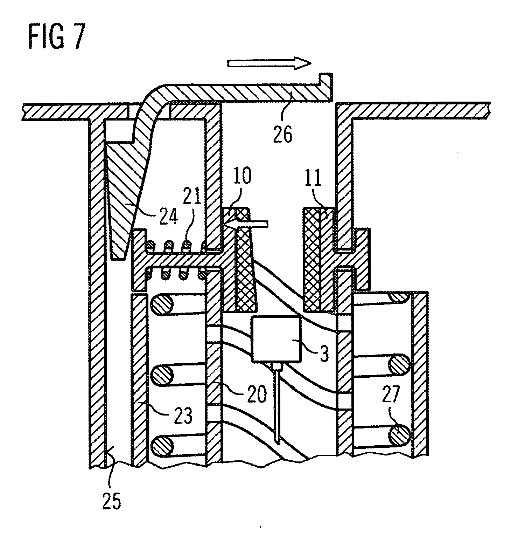

The needle 3 is released when the user pulls the lid 26 out of the bin 1 in order to cover the receiving opening 29. The pushing member 24 is moved from the pushing position to the release position, as shown in figure 7. The first spring 21 relaxes so that the first clamping member 10 is moved radially outwards from the first position to the second position, which causes to the needle 3 to be released. Then the needle 3 drops to the reservoir 7. When the lid 26 is removed again, the needle remover 2 is ready to be used.

Removing the needle by means of the needle remover 2 requires only one hand to operate, which exposes the user to minimal risk of contact with the contaminated needle.

The bin 1 and the needle remover 2 may be formed of plastic or metal. In one embodiment the components of the device are made of different materials, e.g. metal, plastic, glass. In one embodiment the needle remover is mounted to a conventional sharp bin or to any other container, e.g. a glass container having a wide opening. This may be a low cost variant or a re-use option for container declared as garbage.

Other implementations are within the scope of the claims. Elements of different embodiments may be combined to form implementations not specifically described herein.

### Reference numerals

- 1: bin
- 2: needle remover
- 3: needle
- 4: holder
- 5: needle collar
- 6: cannula
- 7: reservoir
- 8: guiding member
- 9: helical structure
- 9a: first helical cut-out
- 9b: second helical cut-out
- 10: first clamping member
- 11: second clamping member
- 12: first jaw member
- 13: first arm
- 14: first protruding member
- 15: second jaw member
- 16: second arm
- 17: second protruding member
- 18: first gripping lining
- 19: second gripping lining
- 20: first sleeve
- 21: first spring
- 22: inserting direction
- 23: second sleeve
- 24: pushing member
- 25: side wall
- 26: lid
- 27: second spring
- 28: stop disc
- 29: top opening
- 30: bottom opening
- 31: gripping means
- 32a: first top end
- 32b: second top end
- 33a: first bottom end
- 33b: second bottom end

## Claims

1. A needle remover (2) for removing a needle (3) from a holder (4), comprising:
a guiding member (8) being suitable for receiving the needle (3), and
a gripping means (31) being suitable for gripping the needle (3) when the needle (3) is at least partly inserted in the guiding member (8) in an inserting direction (22), wherein the gripping means (31) is configured to rotate when the gripping means (31) is moved in the inserting direction (22) with respect to the guiding member (8) so that the gripped needle (2) is unscrewed from the holder (4) when the holder (4) is moved in the inserting direction (22) with respect to the guiding member (8), **characterized in that** the guiding member (8) comprises a helical structure (9), and wherein the gripping means (31) is configured to hold the needle (3) in a force-locked manner.

2. The needle remover (2) according to claim 2, wherein the gripping means (31) is configured to engage with the helical structure (9).

3. The needle remover (2) according to any of the claims 1 or 2, wherein the gripping means (31) is suitable for releasing the needle (3) after unscrewing the needle (3).

4. The needle remover (2) according to any of the claims 1 to 3, wherein the gripping means (31) comprises a first clamping member (10) and a second clamping member (11) which are moveable with respect to one another.

5. The needle remover (2) according to claim 4, wherein the first clamping member (10) is positioned in a first position with respect to the second clamping member (11) when the needle (3) is gripped, and wherein the needle (3) is released when the first clamping member (10) is positioned in a second position with respect to the second clamping member (11).

6. The needle remover according to any of the claims 1 to 5, wherein the guiding member (8) comprises a first sleeve (20) having a helical cut-out (9a).

7. The needle remover according to claim 6, wherein the first clamping member (10) comprises a jaw member (12) which is located inside the first sleeve (20) and an protruding member (14) which protrudes from the first sleeve (20), the first clamping member (10) being radially moveable with respect to the first sleeve (20), and the first clamping (10) member being moveable along the helical cut-out (9a).

8. The needle remover (2) according to claim 7, wherein a biasing member (21) acts normally to urge the first clamping member (10) in the second position.

9. The needle remover (2) according to claim 8, comprising a moveable pushing member (24), wherein the pushing member (24) is configured to push the first clamping member (10) in the first position when the pushing member (24) is positioned in a pushing position, and wherein the biasing member (21) urges the first clamping member (10) in the second position when the pushing member (24) is positioned in a release position.

10. The needle remover (2) according to claim 9, wherein the first sleeve (20) is located at least partly inside a second sleeve (23) so that the first clamping member (10) is positioned in the first position when the gripping means (31) is moved in the inserting direction (22).

11. A bin (1) comprising a needle reservoir (7) which is suitable for receiving unscrewed needles (3) and the needle remover (2) according to any of the previous claims.

12. The bin (1) according to claim 11, comprising a lid (26) which is suitable to cover an opening (29) of the guiding member (8), wherein the lid (26) is coupled with the pushing member (24) so that the pushing member (24) is positioned in the release position when the lid (26) covers the opening (29).

13. A method for removing a needle (3) from a holder (4) by a needle remover (2), comprising:
- inserting the needle (3) into a guiding member (8) of the needle remover (2) in an inserting direction (22),
- gripping the needle (3) by the gripping means (31) in a force-locked manner when the needle (3) is at least partly inserted in the guiding member (8), and
- rotating the gripping means (31) when the gripping means (31) is moved in the inserting direction (22) with respect to the guiding member (8) comprising a helical (9) structure so that the gripped needle (3) is unscrewed from the holder (4) when the holder (4) is moved in the inserting direction (22) with respect to the guiding member (8).

## Patentansprüche

1. Nadelentferner (2) zur Entfernung einer Nadel (3) von einem Halter (4), umfassend:
ein Führungselement (8), das geeignet ist, um die Nadel (3) aufzunehmen, und
ein Greifmittel (31), das geeignet ist, um die Nadel (3) zu ergreifen, wenn die Nadel (3) mindestens teilweise im Führungselement (8) in einer Einführungsrichtung (22) eingeführt ist, wobei das Greifmittel (31) so konfiguriert ist, dass es sich dreht, wenn das Greifmittel (31) in der Einführungsrichtung (22) im Verhältnis zum Führungselement (8) bewegt wird, so dass die ergriffene Nadel (2) vom Halter (4) abgeschraubt wird, wenn der Halter (4) in der Einführungsrichtung (22) im Verhältnis zum Führungselement (8) bewegt wird, **dadurch gekennzeichnet, dass** das Führungselement (8) eine spiralförmige Struktur (9) umfasst, und wobei das Greifmittel (31) so konfiguriert ist, dass es die Nadel (3) in einer kraftschlüssigen Weise hält.

2. Nadelentferner (2) nach Anspruch 1, bei dem das Greifmittel (31) so konfiguriert ist, dass es in die spiralförmige Struktur (9) eingreift.

3. Nadelentferner (2) nach einem der Ansprüche 1 oder 2, bei dem das Greifmittel (31) geeignet ist, um die Nadel (3) nach dem Abschrauben der Nadel (3) freizugeben.

4. Nadelentferner (2) nach einem der Ansprüche 1 bis 3, bei dem das Greifmittel (31) ein erstes Klemmelement (10) und ein zweites Klemmelement (11) umfasst, die im Verhältnis zueinander bewegbar sind.

5. Nadelentferner (2) nach Anspruch 4, bei dem das erste Klemmelement (10) im Verhältnis zum zweiten Klemmelement (11) in einer ersten Position positioniert ist, wenn die Nadel (3) ergriffen wird, und wobei die Nadel (3) freigegeben wird, wenn das erste Klemmelement (10) in einer zweiten Position im Verhältnis zum zweiten Klemmelement (11) positioniert ist.

6. Nadelentferner nach einem der Ansprüche 1 bis 5, bei dem das Führungselement (8) eine erste Hülse (20) mit einem spiralförmigen Ausschnitt (9a) umfasst.

7. Nadelentferner nach Anspruch 6, bei dem das erste Klemmelement (10) ein Backenelement (12), das sich im Inneren der ersten Hülse (20) befindet, sowie ein vorspringendes Element (14) umfasst, das aus der ersten Hülse (20) herausragt, wobei das erste Klemmelement (10) im Verhältnis zur ersten Hülse (20) radial bewegbar und das erste Klemmelement (10) entlang dem spiralförmigen Ausschnitt (9a) bewegbar ist.

8. Nadelentferner (2) nach Anspruch 7, bei dem ein Vorspannelement (21) normalerweise dazu dient, das erste Klemmelement (10) in die zweite Position zu drängen.

9. Nadelentferner (2) nach Anspruch 8, der ein bewegbares Schiebeelement (24) umfasst, wobei das Schiebeelement (24) so konfiguriert ist, dass es das erste Klemmelement (10) in die erste Position schiebt, wenn das Schiebeelement (24) in einer Schiebeposition positioniert ist, und wobei das Vorspannelement (21) das erste Klemmelement (10) in die zweite Position drängt, wenn das Schiebeelement (24) in einer Freigabeposition positioniert ist.

10. Nadelentferner (2) nach Anspruch 9, bei dem sich die erste Hülse (20) mindestens teilweise im Inneren einer zweiten Hülse (23) befindet, so dass das erste Klemmelement (10) in der ersten Position positioniert ist, wenn das Greifmittel (31) in der Einführungsrichtung (22) bewegt wird.

11. Behältnis (1), das ein Nadelreservoir (7) umfasst, das geeignet ist, abgeschraubte Nadeln (3) sowie den Nadelentferner (2) nach einem der vorstehend aufgeführten Ansprüche aufzunehmen.

12. Behältnis (1) nach Anspruch 11, das einen Deckel (26) umfasst, der geeignet ist, um eine Öffnung (29) des Führungselements (8) abzudecken, wobei der Deckel (26) mit dem Schiebeelement (24) gekoppelt ist, so dass das Schiebeelement (24) in der Freigabeposition positioniert ist, wenn der Deckel (26) die Öffnung (29) abdeckt.

13. Verfahren zur Entfernung einer Nadel (3) von einem Halter (4) durch einen Nadelentferner (2), umfassend:
- Einführen der Nadel (3) in ein Führungselement (8) des Nadelentferners (2) in einer Einführungsrichtung (22),
- Ergreifen der Nadel (3) durch das Greifmittel (31) in einer kraftschlüssigen Weise, wenn die Nadel (3) mindestens teilweise im Führungselement (8) eingeführt ist, und
- Drehen des Greifmittels (31), wenn das Greifmittel (31) in der Einführungsrichtung (22) im Verhältnis zum eine spiralförmige (9) Struktur umfassenden Führungselement (8) bewegt wird, so dass die ergriffene Nadel (3) vom Halter (4) abgeschraubt wird, wenn der Halter (4) in der Einführungsrichtung (22) im Verhältnis zum Führungselement (8) bewegt wird.

## Revendications

1. Dispositif d'enlèvement d'aiguille (2) pour enlever une aiguille (3) d'un support (4), comprenant :
un organe de guidage (8) approprié pour recevoir l'aiguille (3), et
un moyen de préhension (31) approprié pour saisir l'aiguille (3) lorsque l'aiguille (3) est au moins en partie insérée dans l'organe de guidage (8) dans une direction d'insertion (22), le moyen de préhension (31) étant configuré pour tourner lorsque le moyen de préhension (31) est déplacé dans la direction d'insertion (22) par rapport à l'organe de guidage (8) de telle sorte que l'aiguille saisie (2) soit dévissée du support (4) lorsque le support (4) est déplacé dans la direction d'insertion (22) par rapport à l'organe de guidage (8), **caractérisé en ce que** l'organe de guidage (8) comprend une structure hélicoïdale (9) et dans lequel le moyen de préhension (31) est configuré pour retenir l'aiguille (3) de manière verrouillée par force.

2. Dispositif d'enlèvement d'aiguille (2) selon la revendication 1, dans lequel le moyen de préhension (31) est configuré pour s'engager avec la structure hélicoïdale (9).

3. Dispositif d'enlèvement d'aiguille (2) selon l'une quelconque des revendications 1 ou 2, dans lequel le moyen de préhension (31) est approprié pour libérer l'aiguille (3) après le dévissage de l'aiguille (3).

4. Dispositif d'enlèvement d'aiguille (2) selon l'une quelconque des revendications 1 à 3, dans lequel le moyen de préhension (31) comprend un premier organe de serrage (10) et un deuxième organe de serrage (11) qui peuvent être déplacés l'un par rapport à l'autre.

5. Dispositif d'enlèvement d'aiguille (2) selon la revendication 4, dans lequel le premier organe de serrage (10) est positionné dans une première position par rapport au deuxième organe de serrage (11) lorsque l'aiguille (3) est saisie, et dans lequel l'aiguille (3) est libérée lorsque le premier organe de serrage (10) est positionné dans une deuxième position par rapport au deuxième organe de serrage (11).

6. Dispositif d'enlèvement d'aiguille selon l'une quelconque des revendications 1 à 5, dans lequel l'organe de guidage (8) comprend un premier manchon (20) ayant une découpure hélicoïdale (9a).

7. Dispositif d'enlèvement d'aiguille selon la revendication 6, dans lequel le premier organe de serrage (10) comprend un organe de mâchoire (12) situé à l'intérieur du premier manchon (20) et un organe saillant (14) faisant saillie depuis le premier manchon (20), le premier organe de serrage (10) pouvant être déplacé radialement par rapport au premier manchon (20), et le premier organe de serrage (10) pouvant être déplacé le long de la découpure hélicoïdale (9a).

8. Dispositif d'enlèvement d'aiguille (2) selon la revendication 7, dans lequel un organe de précontrainte (21) agit normalement de manière à solliciter le premier organe de serrage (10) dans la deuxième position.

9. Dispositif d'enlèvement d'aiguille (2) selon la revendication 8, comprenant un organe de poussée mobile (24), dans lequel l'organe de poussée (24) est configuré pour pousser le premier organe de serrage (10) dans la première position lorsque l'organe de poussée (24) est positionné dans une position de poussée, et dans lequel l'organe de précontrainte (21) sollicite le premier organe de serrage (10) dans la deuxième position lorsque l'organe de poussée (24) est positionné dans une position de libération.

10. Dispositif d'enlèvement d'aiguille (2) selon la revendication 9, dans lequel le premier manchon (20) est situé au moins en partie à l'intérieur d'un deuxième manchon (23) de telle sorte que le premier organe de serrage (10) soit positionné dans la première position lorsque le moyen de préhension (31) est déplacé dans la direction d'insertion (22).

11. Poubelle (1) comprenant un réservoir pour aiguilles (7) approprié pour recevoir des aiguilles dévissées (3) et le dispositif d'enlèvement d'aiguille (2) selon l'une quelconque des revendications précédentes.

12. Poubelle (1) selon la revendication 11, comprenant un couvercle (26) approprié pour recouvrir une ouverture (29) de l'organe de guidage (8), dans laquelle le couvercle (26) est accouplé à l'organe de poussée (24) de telle sorte que l'organe de poussée (24) soit positionné dans la position de libération lorsque le couvercle (26) recouvre l'ouverture (29).

13. Procédé pour enlever une aiguille (3) d'un support (4) par un dispositif d'enlèvement d'aiguille (2), comprenant :
- l'insertion de l'aiguille (3) dans un organe de guidage (8) du dispositif d'enlèvement d'aiguille (2) dans une direction d'insertion (22),
- la préhension de l'aiguille (3) par le moyen de préhension (31) de manière verrouillée par force lorsque l'aiguille (3) est au moins en partie insérée dans l'organe de guidage (8), et
- la rotation du moyen de préhension (31) lorsque le moyen de préhension (31) est déplacé dans la direction d'insertion (22) par rapport à l'organe de guidage (8), comprenant une structure hélicoïdale (9) de telle sorte que l'aiguille saisie (3) soit dévissée du support (4) lorsque le support (4) est déplacé dans la direction d'insertion (22) par rapport à l'organe de guidage (8).
